Europäisches Patentamt

European Patent Office

Office européen des brevets

(19)

(11) Numéro de publication: **0 263 044 B1**

(12) **FASCICULE DE BREVET EUROPEEN**

(45) Date de publication de fascicule du brevet:
**10.07.91**

(51) Int. Cl.5: **C07C 275/62**

(21) Numéro de dépôt: **87420256.7**

(22) Date de dépôt: **24.09.87**

(54) Procédé d'isolement et de purification des polyisocyanates à groupement biuret.

(30) Priorité: **30.09.86 FR 8613783**

(43) Date de publication de la demande:
**06.04.88 Bulletin 88/14**

(45) Mention de la délivrance du brevet:
**10.07.91 Bulletin 91/28**

(84) Etats contractants désignés:
**AT BE CH DE ES FR GB GR IT LI LU NL SE**

(56) Documents cités:

CHEMISTRY AND INDUSTRY, no. 12, 19 juin
1982, pages 394-396, Londres, GB; T.R. BOTT:
"Fundamentals of carbon dioxide in solvent
extraction"

Kirk-Othmer Encyclopedia of Chemical
Technology 3-ième edn, tome supplémentaire pages 872-893

(73) Titulaire: **RHONE-POULENC CHIMIE**
**25, quai Paul Doumer**
**F-92408 Courbevoie Cédex(FR)**

(72) Inventeur: **Blind, André**
**10, rue Martin Basse**
**F-69300 Caluire et Cuire(FR)**
Inventeur: **Robin, Jean**
**21, rue Duguesclin**
**F-69006 Lyon(FR)**

(74) Mandataire: **Vignally, Noel et al**
**Rhône-Poulenc Interservices Service Bre-**
**vets Chimie Centre de Recherches des Car-**
**rières B.P. 62**
**F-69192 Saint-Fons Cédex(FR)**

Il est rappelé que: Dans un délai de neuf mois à compter de la date de publication de la mention de la
délivrance du brevet européen, toute personne peut faire opposition au brevet européen délivré, auprès de
l'Office européen des brevets. L'opposition doit être formée par écrit et motivée. Elle n'est réputée formée
qu'après paiement de la taxe d'opposition (art. 99(1) Convention sur le brevet européen).

## EP 0 263 044 B1

**Description**

La présente invention concerne l'isolement et la purification des polyisocyanates à groupement biuret, et plus particulièrement de ces polyisocyanates préparés à partir d'un diisocyanate aliphatique, cycloaliphatique ou arylaliphatique et d'un agent de biurétisation.

Les polyisocyanates à groupement biuret peuvent être utilisés pour préparer des mousses, des adhésifs et des peintures.

Parmi ces applications, l'utilisation de ces polyisocyanates comme constituants des peintures devient de plus en plus importante, notamment pour l'industrie automobile.

Les pellicules de peintures préparées à partir de polyisocyanates aromatiques jaunissent et se fendillent.

Par contre, les pellicules de peinture préparées à partir de polyisocyanates aliphatique, cycloaliphatiques ou arylaliphatiques conservent leurs propriétés pendant de très longues durées et sont donc particulièrement adaptées pour les véhicules automobiles.

Il existe de nombreux procédé de préparation de polyisocyanates à groupement biuret.

Ainsi par exemple, on peut préparer ces polyisocyanates à partir de diisocyanate et d'eau en opérant en milieu solvant, comme dans le brevet français publié sous le numéro 2.382.468.

On peut aussi préparer ces polyisocyanates à partir d'un diisocyanate et d'eau en présence d'un catalyseur, comme dans la demande de brevet allemand No. 2.308.015.

L'obtention de ces polyisocyanates peut aussi être réalisée par réaction d'un diisocyanate avec un alcool tertiaire (FR 1.475.617) ou une amine (US 3.824.266 ou DE 1.174.759).

D'autres composés tels que l'acide formique, les chlorhydrates, l'hydrogène sulfuré ou des hydrates d'alcools ont également été préconisés comme agents de biurétisation.

Quel que soit l'agent de biurétisation mis en oeuvre, on utilise un excès de diisocyanate, souvent très important.

A la fin de la réaction de biurétisation, il faut donc éliminer l'excès de diisocyanate ainsi que le ou les solvants éventuels.

Pour cela, il faut chauffer le mélange réactionnel final, par exemple dans un évaporateur, ce qui permet d'éliminer la majeure partie du diisocyanate résiduel et des solvants éventuels. Ensuite, il est le plus souvent nécessaire de procéder à un nouveau traitement plus poussé afin de tenter de distiller les dernières traces de diisocyanate. En effet la teneur en diisocyanate monomère dans le polyisocyanate à groupement biuret doit être le plus faible possible, d'une part en raison de la relative toxicité du diisocyanate et, d'autre part, pour éviter d'autres réactions pouvant provoquer des précipités ou une augmentation de viscosité.

Ces traitements d'isolement et de purification des polyisocyanates à groupement biuret conduit à la formation de dépôts solides polymériques sur les parois et sur les différents éléments de l'appareillage utilisé. Il est ainsi nécessaire de procéder périodiquement à des arrêts, afin d'éliminer ces dépôts, d'où pertes de temps et coûts supplémentaires.

La présente invention a pour but d'éviter ces inconvénients majeurs en permettant d'isoler et de purifier les polyisocyanates à groupement biuret préparés selon tout procédé de biurétisation des diisocyanates aliphatiques, cycloaliphatiques et arylaliphatiques.

Plus précisément il s'agit d'un procédé d'isolement et de purification d'un polyisocyanate à groupement biuret préparé par réaction entre au moins un diisocyanate aliphatique, cycloaliphatique ou arylaliphatique et un agent de biurétisation caractérisé en ce que ledit polyisocyanate à groupement biuret brut est traité par le gaz carbonique à l'état liquide ou supercritique.

Les diisocyanates monomères qui peuvent être utilisés pour préparer les polyisocyanates à groupement biuret sont les diisocyanates aliphatiques, les diisocyanates cycloaliphatiques et les diisocyanates arylaliphatiques dont les groupements isocyanates ne sont pas directement liés à un cycle aromatique.

A titre d'exemples non limitatifs de ces diisocyanates monomères, on peut citer :
- le diisocyanato-1,3 propane,
- le diisocyanato-1,4 butane,
- le diisocyanato-1,5 pentane,
- le diisocyanato-1,6 hexane,
- le diisocyanato-1,4 éthyl-2 butane,
- le diisocyanato-1,5 méthyl-2 pentane,
- le diisocyanato-1,6 triméthyl-2,2,4 hexane,
- le diisocyanato-1,6 triméthyl-2,4,4 hexane,
- le diisocyanato-1,2 cyclohexane,

2

- le diisocyanato-1,4 cyclohexane,
- le bis(isocyanatométhyl)-1,2 cyclobutane,
- le bis(isocyanato-4 cyclohexyl)méthane,
- le triméthyl-3,3,5 isocyanatométhyl-5 isocyanato-1 cyclohexane,
- le bis(isocyanatométhyl)-1,4 benzène,
- le bis(isocyanatométhyl)-1,2 benzène.

Les diisocyanates monomères peuvent être utilisés séparément ou sous forme de mélanges de plusieurs d'entre eux.

Ainsi, par exemple, le diisocyanato-1,6 hexane, qui est l'un des diisocyanates monomères préférés, peut être utilisé seul ou en mélanges, notamment avec le diisocyanato-1,5 méthyl-2 pentane et/ou le diisocyanato-1,4 éthyl-2 butane ; des mélanges de ces deux derniers diisocyanates peuvent également être utilisés.

L'agent de biurétisation peut être notamment, comme cela a été indiqué précédemment, un alcool tertiaire, une amine ou de l'eau.

L'eau est l'agent de biurétisation le plus courant.

Lorsque l'eau est l'agent de biurétisation, le rapport molaire diisocyanate monomère/eau peut varier dans de très larges limites.

Cependant comme, d'une part, la masse moléculaire du polyisocyanate obtenu est d'autant plus élevée que le rapport molaire diisocyanate monomère/eau est plus bas et, d'autre part, il n'est pas intéressant économiquement d'avoir un rapport trop élevé, on adopte généralement un rapport molaire diisocyanate monomère/eau compris entre 3 et 40. De préférence ce rapport est de 5 à 15.

Les conditions de température pour la réaction de biurétisation sont bien connues.

Lorsque l'agent de biurétisation est l'eau, elles sont généralement comprises entre 70°C et 200°C.

On peut opérer sans solvant ou en présence d'un ou de plusieurs solvants.

Les solvants utilisables sont très nombreux. On peut citer plus particulièrement ceux qui sont décrits dans le brevet français publié sous le numéro 2.382.468, sans que cela soit limitatif.

La réaction de biurétisation peut également être conduite sous une pression de gaz carbonique supérieure à la pression atmosphérique, par exemple de 1,2 bar à 100 bars et de préférence de 1,5 bar à 50 bars. Dans ce cas on peut opérer en milieu solvant, mais également sans solvant, ce qui permet une meilleure productivité de l'appareillage.

On peut également mettre en oeuvre la réaction de biurétisation en présence d'un acide carboxylique aliphatique, cycloaliphatique, aromatique ou hétérocyclique, ou d'un anhydride de ces acides, comme catalyseur. On peut ainsi par exemple utiliser de 0,005 % à 2,0 % en poids d'acide acétique, d'acide propionique, d'acide butyrique, d'acide isobutyrique ou d'acide benzoïque par rapport au poids du diisocyanate monomère.

On peut également, bien entendu, préparer le biuret en utilisant un catalyseur acide carboxylique, tel qu'indiqué précédemment, et en réalisant la réaction sous pression de gaz carbonique.

L'extraction, après la réaction de biurétisation, de l'excès de diisocyanate et le cas échéant du ou des solvants est faite à l'aide du gaz carbonique à l'état liquide ou à l'état supercritique.

Le gaz carbonique présente l'avantage d'être bon marché et non-toxique.

L'extraction peut se faire en continu ou en discontinu.

A l'état liquide, le gaz carbonique est généralement mis en oeuvre à une température comprise entre 0°C et 31°C et sous une pression de 30 à 500 bars.

Il est préférable d'opérer entre 20°C et 31°C, afin de ne pas avoir une viscosité trop importante du biuret à traiter, et sous une pression de 60 à 300 bars.

A l'état supercritique, la température est supérieure à la température critique du gaz carbonique (31,4°C) et la pression est généralement de 73 à 500 bars. De préférence la pression est de 73 à 350 bars et la température est comprise entre 31,4°C et 100°C.

Il est possible de traiter la masse réactionnelle dès la fin de la réaction de biurétisation.

Cependant, compte-tenu des quantités importantes de diisocyanate monomère en excès et le plus fréquemment du ou des solvants, il peut être préférable et plus économique de procéder préalablement à une élimination rapide de la plus grande partie du diisocyanate monomère en excès et du solvant lorsque ce dernier est présent.

Ce traitement, qui est souvent une évaporation très rapide sous pression réduite, ne nécessite pas de chauffer à une température élevée. Il ne présente donc pas, ou très peu, l'inconvénient mentionné précédemment, c'est-à-dire de provoquer dans l'appareillage un dépôt polymérique nécessitant de fréquents arrêts pour nettoyage.

En pratique donc, la masse obtenue après la réaction de biurétisation est envoyée dans un évaporateur

3

EP 0 263 044 B1

à couche mince, qui permet de séparer la majeure partie du solvant et environ 80 à 90 % du diisocyanate monomère en excès.

Le polyisocyanate à groupement biuret, contenant une quantité encore relativement importante de diisocyanate monomère et éventuellement des traces de solvant, est alors traité à l'aide de $CO_2$ liquide ou de $CO_2$ à l'état supercritique.

On peut opérer en discontinu, par exemple en mélangeant dans un réacteur le polyisocyanate à groupement biuret que l'on veut purifier avec le gaz carbonique liquide ou à l'état supercritique que l'on y a introduit.

Après séparation du gaz carbonique contenant les éventuelles traces de solvants, le diisocyanate monomère et le dimère éventuellement présent, du polyisocyanate à groupement biuret purifié, on peut séparer ledit gaz carbonique des extraits par détente et/ou augmentation de température.

L'extraction peut être conduite de manière conventionnelle, dans un appareillage connu en soi.

A partir d'une source de gaz carbonique, on envoie ledit gaz dans un échangeur thermique où il est liquéfié ; il est ensuite véhiculé, à la pression désirée, à l'aide d'une pompe, vers un autre échangeur thermique d ans lequel il est porté à la température choisie pour l'extraction.

Ledit gaz d'extraction, à l'état liquide ou à l'état supercritique, est ensuite envoyé dans l'appareil d'extraction, qui peut être par exemple une colonne remplie d'un garnissage permettant un meilleur contact entre le polyisocyanate à groupement biuret et le gaz carbonique.

Le polyisocyanate à groupement biuret peut être introduit à l'autre extrêmité de la colonne : on a alors une extraction à contre-courant. Moins fréquemment, on peut l'introduire à la même extrêmité que le gaz carbonique : on a alors une extraction à co-courant.

Le polyisocyanate à groupement biuret purifié est récupéré à une extrêmité de la colonne d'extraction, tandis que le gaz carbonique, chargé du diisocyanate monomère, du dimère de ce diisocyanate éventuellement présent dans le polyisocyanate et des éventuelles traces de solvant restant, est soumis à un traitement pour le séparer des composés extraits.

On peut pour cela agir soit par diminution de sa pression, soit par augmentation de sa température, soit à la fois par diminution de sa pression et augmentation de sa température. Ces opérations ont pour but de modifier le pouvoir solvant du gaz carbonique.

La diminution de pression, ou détente, peut se faire en une ou plusieurs étapes et le gaz carbonique peut être détendu jusqu'a une pression égale à la pression atmosphérique ou jusqu'à une pression plus élevée , sous laquelle il sera recyclé dans le cas d'un procédé continu.

En effet, si ledit gaz d'extraction est recyclé, il est économiquement préférable de ne pas le détendre jusqu'à la pression atmosphérique, ce qui nécessiterait une plus grande dépense d'énergie pour le recomprimer dans le cycle suivant du procédé. Il est préférable de le détendre que jusqu'à une pression sous laquelle les composés extraits ne sont pas, ou très peu, solubles.

Comme cela a été indiqué précédemment, le procédé peut être mis en oeuvre de manière continue ou discontinue et l'appareillage utilisé n'est pas limité à la description du principe de fonctionnement décrit ci-avant.

Généralement, dans le cadre du procédé de l'invention, on préfère mettre en oeuvre le gaz carbonique à l'état supercritique.

Les exemples qui suivent illustrent la présente invention.


EXEMPLE 1

Dans une colonne d'extraction en acier inoxydable de 19 mm de diamètre et de 250 mm de hauteur, remplie de garnissage Dixon, on introduit 85 g de polyisocyanate à groupement biuret préparé à partir de diisocyanato-1,6 hexane (HDI). La teneur de ce polyisocyanate en HDI libre est de 8,8 % en poids.

La colonne est thermostatée à 20°C. On admet le gaz carbonique à l'état liquide jusqu'à une pression de 100 bars.

La pression de détente de la colonne d'extraction est maintenue à la valeur désirée par un régulateur de pression.

Lorsque le système est en équilibre, on fait passer par le bas de la colonne le gaz carbonique à l'état liquide sous un débit de 1600 grammes/heure environ.

Les extraits sont récupérés, au delà du régulateur de pression, par détente à pression atmosphérique, dans une succession de flacons.

La teneur en HDI du polyisocyanate traité est contrôlée par chromatographie en phase liquide. Le tableau (I) ci-après rassemble les résultats de l'essai :
- la quantité d'extrait (HDI + dimère de l'HDI)

4

EP 0 263 044 B1

- le rapport pondéral extrait/polyisocyanate chargé
- la teneur en HDI du polyisocyanate traité

en fonction de la quantité de $CO_2$ liquide engagée.

## Tableau (I)

| Poids cumulé de $CO_2$ liquide utilisé | Poids cumulé d'extrait | Extrait/ polyisocyanate chargé | Teneur en HDI dans le poly- isocyanate traité |
|---|---|---|---|
| 400 g | 4,7 g | 5,5 % en poids | - |
| 800 g | 6,8 g | 8,0 % en poids | 0,17 % en poids |
| 1600 g | 9,8 g | 11,5 % en poids | 0,03 % en poids |

## EXEMPLE 2

Dans la colonne décrite dans l'exemple 1, on introduit 86 g de polyisocyanate à groupement biuret préparé à partir d'HDI (teneur en HDI libre : 8,8 % en poids).

La colonne étant thermostatée à 40°C, on admet le gaz carbonique à l'état supercritique jusqu'à une pression de 200 bars.

La pression de détente est maintenue à la valeur désirée au moyen d'un régulateur de pression.

Lorsque le système est en équilibre, on fait circuler par le bas de la colonne le $CO_2$ à l'état supercritique, avec un débit moyen de 1600 grammes/heure environ.

Les extraits sont récupérés dans les conditions décrites dans l'exemple 1.

Les résultats obtenus sont rassemblés dans le tableau (II) :

5

EP 0 263 044 B1

| Poids cumulé de $CO_2$ supercritique utilisé | Poids cumulé d'extrait | Extrait/ polyisocyanate chargé | Teneur en HDI dans le poly- isocyanate traité |
|---|---|---|---|
| 200 g | 4,4 g | 5,1 % en poids | - |
| 600 g | 9,2 g | 10,7 % en poids | 0,04 % en poids |
| 1000 g | 11,0 g | 12,8 % en poids | 0,015 % en poids |

EXEMPLE 3

On répète l'exemple 2 dans les mêmes conditions (200 bars, 40°C), mais en fin d'extraction les extraits sont récupérés par détente du $CO_2$ à 60 bars et 40°C.

Le gaz carbonique séparé des extraits est reliquéfié et recyclé, après compression, dans la colonne d'extraction pour l'extraction d'une nouvelle quantité de polyisocyanate à groupement biuret.

Le poids d'extraits récupéré est de 10 g, ce qui représente 11,6 % en poids du polyisocyanate chargé.

La teneur en HDI libre du polyisocyanate traité lors du premier cycle d'extraction est de 0,005 % en poids en fin d'essai.

**Revendications**

1. Procédé d'isolement et de purification de polyisocyanates à groupement biuret, préparés à partir d'au moins un diisocyanate aliphatique, cycloaliphatique ou arylaliphatique et d'un agent de biurétisation, caractérisé en ce que ledit polyisocyanate à groupement biuret brut est traité par le gaz carbonique à l'état liquide ou supercritique.

2. Procédé selon la revendication 1, caractérisé en ce que le polyisocyanate à groupement biuret est préparé à partir d'au moins un diisocyanate monomère choisi dans le groupement comprenant :
   - le diisocyanato-1,3 propane,
   - le diisocyanato-1,4 butane,
   - le diisocyanato-1,5 pentane,
   - le diisocyanato-1,6 hexane,
   - le diisocyanato-1,4 éthyl-2 butane,
   - le diisocyanato-1,5 méthyl-2 pentane,
   - le diisocyanato-1,6 triméthyl-2,2,4 hexane,
   - le diisocyanato-1,6 triméthyl-2,4,4 hexane,
   - le diisocyanato-1,2 cyclohexane,
   - le diisocyanato-1,4 cyclohexane,
   - le bis(isocyanatométhyl)-1,2 cyclobutane,
   - le bis (isocyanato-4 cyclohexyl)méthane,
   - le triméthyl-3,3,5 isocyanatométhol-5 isocyanato-1 cyclohexane,

6

- le bis(isocyanatométhyl)-1,4 benzène,
- le bis(isocyanatométhyl)-1,2 benzène.

3. Procédé selon l'une des revendications 1 ou 2, caractérisé en ce que le polyisocyanate à groupement biuret est préparé à partir de diisocyanato-1,6 hexane seul ou en mélange avec le diisocyanato-1,5 méthyl-2 pentane et/ou le diisocyanato-1,4 éthyl-2 butane.

4. Procédé selon l'une des revendications 1 à 3, caractérisé en ce que l'agent de biurétisation utilisé est l'eau.

5. Procédé selon la revendication 4, caractérisé en ce que le polyisocianate biuret est préparé en utilisant un rapport molaire diisocyanate monomère/eau compris entre 3 et 40 et de préférence entre 5 et 15.

6. Procédé selon l'une des revendications 1 à 5, caractérisé en ce que le gaz carbonique est utilisé à l'état supercritique.

7. Procédé selon la revendication 1, caractérisé en ce que l'on opère à une température comprise entre 0 et 31°C et sous une pression de 30 à 500 bars;

8. Procédé selon l'une des revendications 1 et 7, caractérisé en ce que l'on opère à une température comprise entre 20 et 31°C et sous une pression de 60 à 300 bars.

9. Procédé selon la revendication 1, caractérisé en ce que l'on opère à une température supérieure à la température critique du gaz carbonique de préférence comprise entre 31°C et 100°C et sous une pression de 73 à 500 bars et de préférence de 73 à 350 bars.

10. Procédé selon l'une des revendications 1 à 9, caractérisé en ce que l'on élimine, préalablement à l'extraction par 1e gaz carbonique à l'état liquide ou supercritique, la plus grande partie de l'excès de diisocyanate monomère et du solvant lorsque ce dernier est présent.

## Claims

1. Process for isolating and purifying polyisocyanates containing a biuret group, prepared from at least one aliphatic, cycloaliphatic or arylaliphatic diisocyanate and a biuret-forming agent, characterised in that the crude said polyisocyanate containing a biuret group is treated with carbon dioxide in the liquid or supercritical state.

2. Process according to Claim 1, characterised in that the polyisocyanate containing a biuret group is prepared from at least one diisocyanate monomer chosen from the group comprising:
   1,3-diisocyanatopropane,
   1,4-diisocyanatobutane,
   1,5-diisocyanatopentane,
   1,6-diisocyanatohexane,
   1,4-diisocyanato-2-ethylbutane,
   1,5-diisocyanato-2-methylpentane,
   1,6-diisocyanato-2,2,4-trimethylhexane,
   1,6-diisocyanato-2,4,4-trimethylhexane,
   1,2-diisocyanatocyclohexane,
   1,4-diisocyanatocyclohexane,
   1,2-bis(isocyanatomethyl)cyclobutane,
   bis(4-isocyanatocyclohexyl)methane,
   3,3,5-trimethyl-5-isocyanatomethyl-
   1-isocyanatocyclohexane,
   1,4-bis(isocyanatomethyl)benzene,
   1,2-bis(isocyanatomethyl)benzene,

3. Process according to one of Claims 1 and 2, characterised in that the polyisocyanate containing a biuret group is prepared from 1,6-diisocyanatohexane alone or in the form of a mixture with 1,5-

7

EP 0 263 044 B1

diisocyanato-2-methylpentane and/or 1,4-diisocyanato-2-ethylbutane.

4. Process according to one of Claims 1 to 3, characterised in that the biuret-forming agent used is water.

5. Process according to Claim 4, characterised in that the biuret polyisocyanate is prepared using a diisocyanate monomer/water mole ratio of between 3 and 40, and preferably between 5 and 15.

6. Process according to one of Claims 1 to 5, charaterised in that carbon dioxide is used in the supercritical state.

7. Process according to Claim 1, characterised in that the treatment is performed at a temperature of between 0 amd 31 ° C and under a pressure of 30 to 500 bars.

8. Process according to one of Claims 1 and 7, characterised in that the treatment is performed at a temperature of between 20 and 31 ° C and under a pressure of 60 to 300 bars.

9. Process according to Claim 1, characterised in that the treatment is performed at a temperature above the critical temperature of carbon dioxide, preferably between 31 ° C and 100 ° C, and under a pressure of 73 to 500 bars and preferably from 73 to 350 bars.

10. Process according to one of Claims 1 to 9, characterised in that, prior to the extraction with carbon dioxide in the liquid or supercritical state, most of the excess diisocyanate monomer, and of the solvent when the latter is present, is removed.

## Ansprüche

1. Verfahren zum Isolieren und Reinigen von Polyisocyanaten mit Biuretgruppe, hergestellt ausgehend von mindestens einem aliphatischen, cycloaliphatischen oder arylaliphatischen Diisocyanat und einem biuretbildenden Mittel, dadurch gekennzeichnet, daß das rohe Polyisocyanat mit Biuretgruppe mit Kohlendioxid in flüssigem oder superkritischem Zustand behandelt wird.

2. Verfahren nach Anspruch 1, dadurch gekennzeichnet, daß das Polyisocyanat mit Biuretgruppe ausgehend von mindestens einem monomeren Diisocyanat hergestellt wird, das aus der folgenden Gruppe ausgewählt wird:

Propan-1,3-diisocyanat,
Butan-1,4-diisocyanat,
Pentan-1,5-diisocyanat,
Hexan-1,6-diisocyanat,
2-Ethylbutan-1,4-diisocyanat,
2-Methylpentan-1,5-diisocyanat,
2,2,4-Trimethylhexan-1,6-diisocyanat,
2,4,4-Trimethylhexan-1,6-diisocyanat,
Cyclohexan-1,2-diisocyanat,
Cyclohexan-1,4-diisocyanat,
1,2-Bis(isocyanatomethyl)-cyclobutan,
Bis(4-isocyanatocyclohexyl)-methan,
3,3,5-Trimethyl-5-isocyanatomethyl-cyclohexan-1-isocyanat,
1,4-Bis(isocyanatomethyl)-benzol,
1,2-Bis(isocyanatomethyl)-benzol.

3. Verfahren nach einem der Ansprüche 1 oder 2, dadurch gekennzeichnet, daß das Polyisocyanat mit Biuretgruppe ausgehend von Hexan-1,6-diisocyanat alleine oder im Gemisch mit 2-Methyl-pentan-1,5-diisocyanat und/oder 2-Ethylbutan-1,4-diisocyanat hergestellt wird.

4. Verfahren nach einem der Ansprüche 1 bis 3, dadurch gekennzeichnet, daß das eingesetzte biuretbildende Mittel Wasser ist.

8

5. Verfahren nach Anspruch 4, dadurch gekennzeichnet, daß das Polyisocyanat-biuret unter Anwendung eines Molverhältnisses von monomerem Diisocyanat zu Wasser im Bereich von 3 bis 40 und vorzugsweise von 5 bis 15 hergestellt wird.

6. Verfahren nach einem der Ansprüche 1 bis 5, dadurch gekennzeichnet, daß das Kohlendioxid in superkritischem Zustand eingesetzt wird.

7. Verfahren nach Anspruch 1, dadurch gekennzeichnet, daß man bei einer Temperatur im Bereich von 0 bis 31°C und unter einem Druck von 30 bis 500 bar arbeitet.

8. Verfahren nach einem der Ansprüche 1 und 7, dadurch gekennzeichnet, daß man bei einer Temperatur im Bereich von 20 bis 31°C und unter einem Druck von 60 bis 300 bar arbeitet.

9. Verfahren nach Anspruch 1, dadurch gekennzeichnet, daß man bei einer Temperatur oberhalb der kritischen Temperatur von Kohlendioxid, vorzugsweise im Bereich von 31°C bis 100°C und unter einem Druck von 73 bis 500 bar, vorzugsweise von 73 bis 300 bar, arbeitet.

10. Verfahren nach einem der Ansprüche 1 bis 9, dadurch gekennzeichnet, daß man vor der Extraktion mit Kohlendioxid in flüssigem oder superkritischem Zustand den größten Teil des überschüssigen monomeren Diisocyanats sowie des Lösungsmittels, wenn dieses letztere vorhanden ist, entfernt.